(19) **Europäisches Patentamt**

**European Patent Office**

**Office européen des brevets**

(11) Veröffentlichungsnummer: **0 297 390 B1**

(12) ## EUROPÄISCHE PATENTSCHRIFT

(45) Veröffentlichungstag der Patentschrift: **16.09.92**

(21) Anmeldenummer: **88109828.9**

(22) Anmeldetag: **21.06.88**

(51) Int. Cl.⁵: **G01N 33/52**, G01N 31/22

Die Akte enthält technische Angaben, die nach dem Eingang der Anmeldung eingereicht wurden und die nicht in dieser Patentschrift enthalten sind.

(54) Verfahren zur Herstellung eines diagnostischen Testträgers und entsprechender Testträger.

(30) Priorität: **27.06.87 DE 3721236**

(43) Veröffentlichungstag der Anmeldung:
**04.01.89 Patentblatt 89/01**

(45) Bekanntmachung des Hinweises auf die Patenterteilung:
**16.09.92 Patentblatt 92/38**

(84) Benannte Vertragsstaaten:
**AT BE CH DE ES FR GB GR IT LI LU NL SE**

(56) Entgegenhaltungen:
EP-A- 0 045 476          EP-A- 0 158 190
EP-A- 0 164 960          EP-A- 0 166 365
EP-A- 0 287 883          EP-A- 0 290 921
AT-B- 360 961            GB-A- 2 154 737
US-A- 3 993 451          US-A- 4 258 001

HANDBOOK OF ADHESIVES, Robert E. Krieger Publishing Company, New York, NY (US); p. 447

I.KATZ, Adhesive Materials; pp. 44-45

C.V.CAGLE, Adhesive Bonding; pp. 18-19

(73) Patentinhaber: **BOEHRINGER MANNHEIM GMBH**
**Sandhofer Strasse 116**
**W-6800 Mannheim 31(DE)**

(72) Erfinder: **Macho, Heinz Kurt**
**Ortsstr. 42**
**W-6149 Fürth/Fahrenbach(DE)**
Erfinder: **Hungenberg, Klaus Dieter, Dipl.-Chem.Dr.**
**Ortsstr. 135**
**W-6343 Birkenau-Hornbach(DE)**

(74) Vertreter: **Pfeifer, Hans-Peter, Dr.rer.nat.**
**Patentanwalt Nowackanlage 15**
**W-7500 Karlsruhe 1(DE)**

**Beschreibung**

Die Erfindung betrifft ein Verfahren zur Herstellung eines diagnostischen Testträgers zur Untersuchung einer Probenflüssigkeit, welches eine Verfahrensschritt einschließt, bei dem zwei für die Probenflüssigkeit saugfähige Schichtmaterialien derartig miteinander verbunden werden, daß die Probenflüssigkeit durch die Verbindungsfläche zwischen den Schichten hindurch aus der einen Schicht in die andere Schicht übertreten kann. Weiter richtet sich die Erfindung auf eine entsprechenden Testträger.

Zur qualitativen oder quantitativen analytischen Bestimmung von Bestandteilen von Körperflüssigkeiten, insbesondere Blut, werden in jüngerer Zeit zunehmend sogenannte trägergebundene Tests verwendet. Bei diesen sind Reagenzien in entsprechenden Schichten eines festen Testträgers eingebettet, der mit der Probe in Kontakt gebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann.

Testträger sind häufig als Teststreifen ausgebildet, die im wesentlichen aus einer länglichen Tragschicht aus Kunststoffmaterial und darauf angebrachten Testschichten bestehen. Es sind jedoch auch Testträer bekannt, die als quadratische oder rechteckige Plättchen gestaltet sind.

Bekannte Testträger enthalten häufig mehrere Schichten, die für die Probenflüssigkeit (dieser Begriff wird hier so verstanden, daß er auch eine aus der Probe abgeleitete Flüssigkeit umfaßt) saugfähig sind. Häufig ist es zweckmäßig, diese Schichten übereinander so anzuordnen, daß die Flüssigkeit aus einer Schicht durch die Verbindungsfläche zwischen zwei Schichten hindurch in die nächst benachbarte saugfähige Schicht eindringt.

Ein derartiger Schichtverbund benachbarter saugfähiger Schichten ist bisher allerdings nur in eingeschränktem Umfang möglich, weil es an geeigneten Verfahren fehlt, die saugfähigen Schichten entsprechend zu montieren.

Problemlos ist der Verbund mehrerer Schichten, wenn die obere Schicht auf die darunterliegende Schicht durch Flüssigbeschichten aufgebracht wird (z.B. EP-A-0164960). Dieses Verfahren stellt jedoch spezielle Anforderungen an die Schichtmaterialien.

Bei Urinteststreifen werden derartige Verbindungen häufig dadurch bewerkstelligt, daß mehrere Testschichten gemeinsam mit einem feinmaschigen Netz überspannt werden, welches seitlich von den Schichten auf dem Trägermaterial des Teststreifens befestigt ist. Diese Befestigungsart geht auf die DE-A 21 18 455 bzw. die entsprechende US-A 3 802 842 zurück. Sie ist für die Analyse kleiner Probenmengen, wie sie bei Blutuntersuchungen verwendet werden, jedoch nicht gut geeignet.

Bei einem anderen bekannten Verfahren werden zwei oder mehrere Testschichten mit Hilfe eines seitlich angeordneten Schmelzkleberstreifens verbunden (s. z.B. EP-A-0045476 und EP-A-290921, letzteres Dokument ist Literatur im Sinne ven Art. 54(3)EPÜ). Dieses Verfahren ist jedoch nur möglich, wenn auf der entsprechenden Seite eine Möglichkeit zur Anbringung des Schmelzkleberstreifens gegeben ist.

In der zuletzt genannten Schrift werden auch Testschichten erwähnt, die aus einem Verbund von miteinander verklebten Partikeln bestehen. Diese sind auch aus anderen Informationsquellen, beispielsweise der EP-A-0164960 und der darin zitierten US-A-4 258 001 bekannt. Der Verbund der Partikel wird dabei dadurch erreicht, daß diese zusammen mit einem Polymer in einer Trägerflüssigkeit dispergiert und auf einer Fläche ausgestrichen werden. Beim Verdampfen der Trägerflüssigkeit und der damit verbundenen Annäherung der Teilchen bildet sich die Partikelverbundstruktur. Dieses Verfahren ist jedoch zur Verbindung von zwei getrennt hergestellten saugfähigen Testschichten ungeeignet. Gleiches gilt auch fur die AT-B-360 961, bei der der Verbund innerhalb einer Testschicht mit Hilfe einer Kleberlösung oder -emulsion verfestigt wird.

In der EP-A 0 166 365 wird die Möglichkeit erwähnt, zwei mikroporöse Schichten eines Testträgers mit Hilfe eines hitzeempfindlichen Klebstoffes (heat-sensitive adhesive) zu verbinden. Diese Anregung bezieht sich auf hitzehärtbare Klebstoffe (Thermosets). Die Möglichkeit, Schmelzkleber zu verwenden, wird nicht erwähnt.

EP-A-0 287 883 (Literatur gemäß Artikel 54(3)EPÜ) zeigt ein Analyseelement in Form eines Teststreifens, bei dem die Zuführung eines definierten Probenvolumens zu einem Reagenzfeld dadurch erreicht wird, daß sich darunter ein Spalt befindet. Der zur Sicherstellung des Spaltes erforderliche Abstand wird durch eine Abstandhalterschicht gewährleistet, die u.a. aus einem thermoplastischen Material bestehen kann, welches bei Erhitzung benutzt werden soll, um die Reagenzschicht mit der tragenden Basisschicht zu verbinden.

Neuerdings ist in der EP-A-208 952 vorgeschlagen worden, mehrere übereinander angeordnete Testfelder miteinander zu vernähen. Dieses Verfahren hat zwar erhebliche Verbesserungen gebracht, führte jedoch zu einer Erhöhung der Herstellkosten und ist nicht in jeder Situation einsetzbar.

Der Erfindung liegt daher die Aufgabe zugrunde, ein Verfahren und einen entsprechenden Testträger zur Verfügung zu stellen, mit dem saugfähige Testträgerschichten einerseits zuverlässig und andererseits rationell so miteinander verbunden werden können, daß ein Flüssigkeitsübertritt durch die Verbindungsfläche hindurch von der einen Schicht in die andere möglich ist. Dabei sollen die Kanten der Schichten nicht zur Verbindung herangezogen werden.

Diese Aufgabe wird bei einem Verfahren der eingangs bezeichneten Art dadurch gelöst, daß ein Schmelzkleber in Form einer unterbrochenen Beschichtung auf das erste Schichtmaterial aufgebracht und das zweite Schichtmaterial, während der Schmelzkleber ausreichend heiß ist, um bindefähig zu sein, angedrückt wird, wobei die Menge des Schmelzklebers und der Anpreßdruck unter Berücksichtigung der Oberflächeneigenschaften der zu verbindenden Schichten so aufeinander abgestimmt sind, daß auch nach dem Aufbringen und Andrücken des zweiten Schichtmaterials keine durchgängige flüssigkeitsundurchlässige Schmelzkleberschicht entsteht.

Der entsprechende Testträger ist durch die Merkmale des Anspruchs 9 gekennzeichnet.

Als Verbindungsfläche im Sinne der Erfindung wird die Fläche angesehen, auf der die beiden Schichten zueinander in einem derartig engen Kontakt stehen, daß ein Flüssigkeitsübertritt von der einen Schicht in die andere möglich ist. Selbstverständlich können die Schichten jeweils größer als die Verbindungsfläche sein, wobei Teile der Schichten dann nur einen losen oder gar keinen Kontakt zueinander haben, so daß in diesen Bereichen kein Flüssigkeitsübertritt möglich ist.

Als Schmelzkleber können handelsübliche Produkte, beispielsweise auf Basis von Ethylen-Vinylacetat-Copolymeren, Polyestern oder Polyamiden zum Einsatz kommen. Derartige Schmelzkleber wurden auch bisher schon bei der Herstellung von diagnostischen Testträgern verwendet, wobei sie vollflächig auf die eine zu verbindende Schicht aufgetragen wurden und die andere Schicht gegen die Schmelzkleberoberfläche gepreßt wurde.

Sämtliche bekannten Schmelzklebermaterialien sind hydrophob. Dies dürfte ein wichtiger Grund sein, warum die Fachwell bisher davor zurückschreckte, eine unterbrochene Schmelzkleberoberschicht zur Verbindung zweier saugfähiger Testträgerschichten einzusetzen. Man war wohl der Auffassung, daß bei zu geringer aufgetragener Schmelzklebermenge keine ausreichend zuverlässige Verbindung erreicht werde bzw. daß bei einem entsprechend erhöhten Schmelzkleberauftrag kein zuverlässiger Flüssigkeitsübertritt von einer Schicht in die andere möglich sei.

Überraschenderweise hat sich gezeigt, daß bei entsprechender Abstimmung der Schmelzklebermenge und des Anpreßdrucks unter Berücksichtigung der Oberflächeneigenschaften der zu verbindenden Schichten beide Forderungen miteinander verbunden werden können. Die genauen Verfahrensbedingungen können auf Basis der vorliegenden Erfindung und der weiter unten gegebenen Hinweise festgelegt werden.

Der Auftrag des Schmelzklebers geschieht bevorzugt entweder durch Aufsprühen oder mittels einer Drucktechnik.

Das Aufsprühen geschieht mit Hilfe einer Sprühvorrichtung, welcher der erhitzte Schmelzkleber und ein Gas, insbesondere Luft, zugeführt wird. Dabei entsteht ein Gewirr von fadenförmigem Schmelzkleber. Obwohl dieses Gewirrr bei den angegebenen notwendigen Schmelzklebermengen eine erhebliche Dichte erreicht, hat sich gezeigt, daß der Flüssigkeitsübertrill von einer Schicht in die andere dadurch nicht in einem praktisch störenden Ausmaß behindert wird. Hierfür ist es vorteilhaft, wenn die aufgebrachte Schmelzklebermenge zwischen 2 und 50 g/m$^2$, besonders bevorzugt zwischen 10 und 25 g/m$^2$ und der Anpreßdruck beim Zusammendrücken der beiden Schichtmaterialien zwischen 0,2 bar und 2,0 bar liegt.

Bei Anwendung einer Drucktechnik wird der Schmelzkleber in Form von die Verbindungsfläche nur teilweise bedeckenden Bereichen, zwischen denen sich schmelzkleberfreie Bereiche befinden, auf das erste Schichtmaterial aufgebracht.

Die Bereiche können grundsätzlich Streifen oder anders geformte flächenstücke sein, wobei die dazwischen liegenden, nicht mit Schmelzkleber beschichteten Flächen groß genug sind, daß die Flüssigkeit hindurchtreten kann. Bevorzugt bedecken die Bereiche zwischen 10 und 80%, besonders bevorzugt zwischen 20 und 50% der Verbindungsfläche.

Eine besonders zuverlässige Verbindung und sogleich ein guter Flüssigkeitsübertritt von einer Schicht in die andere wird erreicht, wenn der Schmelzkleber in Form von sehr kleinen Bereichen, die als Partikel bezeichnet werden aufgebracht wird. Die Partikel sind vorzugsweise über die gesamte Verbindungsfläche verteilt, wobei die Verteilung pro Flächeneinheit nicht notwendigerweise gleichmäßig sein muß. Bevorzugt haben die Schmelzkleberpartikel eine mittlere Flächenausdehnung von höchsten 1,0 mm$^2$, besonders bevorzugt höchstens 0,3 mm$^2$.

Als Drucktechniken kommen insbesondere der Siebdruck oder das Auftragen mit Hilfe eines Radauftragswerks in Betracht.

Die Erfindung wird im folgenden anhand von in den Figuren schematisch dargestellten Ausführungsbeispielen näher erläutert. Es zeigen:

Fig. 1      eine perspektivische Ansicht eines erfindungsgemäßen Testträgers,

Fig. 2      einen Querschnitt entlang der Linie II-II von Fig. 1,

Fig. 3      eine perspektivische Darstellung eines erfindungsgemäßen Sprühverfahrens.

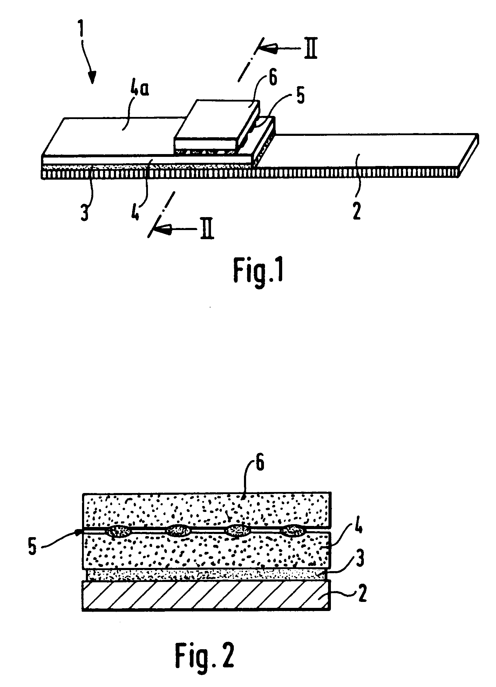

Der in Fig. 1 und 2 dargestellte Testträger 1 hat eine Tragschicht 2, die üblicherweise aus einer Kunststoffolie besteht. Auf der Tragschicht 2 ist mit Hilfe einer durchgehenden Schmelzkleberschicht 3 eine erste saugfähige Schicht 4 befestigt.

Darauf wiederum ist mit Hilfe einer unterbrochenen, flüssigkeitsdurchlässigen Schmelzkleberschicht 5 eine zweite saugfähige Schicht 6 befestigt. Die unterbrochene flüssigkeitsdurchlässige Schmelzkleberschicht 5 ist in den Figuren stark vergröbert in Form verhältnismäßig großer Punkte dargestellt. Bevorzugt besteht sie im Falle des Auftrage mit dem Sprühverfahren aus einem Gewirr sehr dünner Fäden und im Falle des Auftrags mit einer Drucktechnik aus einer sehr großen Anzahl sehr kleiner, über die Verbindungsfläche zwischen den Schichten 4 und 6 verteilter Schmelzkleberpartikel, die als runde Punkte aber auch beispielsweise als Quadrate oder Vielecke geformt sein können.

Fig. 1 zeigt lediglich prinzipiell einen Schichtaufbau, wie er als Konstruktionselement in verschiedenen Versionen von Testträgern eingesetzt werden kann. So kann beispielsweise die saugfähige Schicht 4 als Transportschicht verwendet werden, auf die die Probenflüssigkeit in dem von der zweiten Schicht 6 nicht bedeckten Bereich 4a der Schicht 4 aufgegeben werden kann. Von dort wird sie unter die Schicht 6 transportiert. Die zweite saugfähige Schicht 6 kann in diesem Falle beispielsweise eine Farbreaktionsschicht sein, in der eine zu einem Farbumschlag führende Reaktion stattfindet, die anschließend ausgewertet werden kann. Hierfür ist es wichtig, daß die Probenflüssigkeit aus der Schicht 4 gleichmäßig und ausreichend schnell in die Schicht 6 übertritt, wie dies durch die vorliegende Erfindung erreicht wird.

In einem anderen Fall kann die Probe unmittelbar auf die Schicht 6 aufgegeben werden, wo sie sich verteilt. Ein Probenüberschuß wird in diesem Fall durch die erste Schicht 4 abgesaugt. Auch hier ist ein guter Flüssigkeitsübertritt zwischen den Schichten wichtig.

Die Erfindung erfaßt selbstverständlich auch Fälle, bei denen die beiden saugfähigen Schichten eine ausreichende Festigkeit haben, um ohne zusätzliche Trägerschicht 2 verwendet werden zu können. Die dargestellte Konstruktion, bei der die erste saugfähige Schicht ihrerseits vollflächig auf einen Träger aufgeklebt ist, ist jedoch besonders bevorzugt.

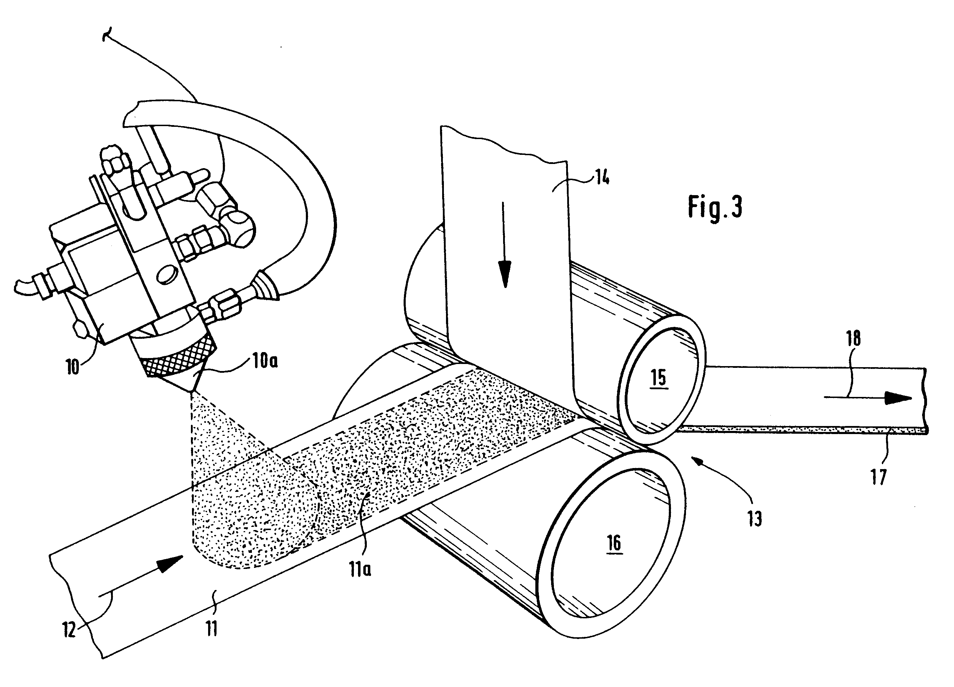

Bei dem in Fig. 3 dargestellten Sprühverfahren wird der Schmelzkleber mit Hilfe einer Sprühvorrichtung 10 auf eine Bahn eines ersten Schichtmaterials 11 aufgesprüht, welches in Richtung des Pfeiles 12 unter der Sprühvorrichtung 10 hindurch einer insgesamt mit 13 bezeichneten Andrückeinrichtung zugeführt wird. Der aufgesprühte Schmelzkleber bildet eine unterbrochene Beschichtung 11 a.

Der Andrückeinrichtung 13 wird außerdem eine Bahn eines zweiten Schichtmaterials 14 zugeführt. Die Walzen 15 und 16 der Andrückeinrichtung 13 werden mit einem einstellbaren Anpreßdruck gegeneinander gedrückt. Dadurch läßt sich die Kraft einstellen, mit der die beiden Schichtmaterialien 11 und 14 in dem Spalt zwischen den Walzen 15 und 16 gegeneinander gedrückt werden.

Beim Andrücken werden die Schictmaterialien 11 und 14 zu dem fertigen Schichtverbund 17 verklebt, der die Andrückeinrichtung 13 in Richtung des Pfeils 18 verläßt.

Wie erwähnt, ist das Aufsprühen von Schmelzkleber auf eine zu verklebende Materialfläche an sich bekannt. Ein entsprechender Sprühkopf wird beispielsweise von der Firma Meltex, Lüneburg, Bundesrepublik Deutschland vertrieben. Es ist daher nicht notwendig, die Sprühvorrichtung 10 näher zu beschreiben.

Wichtig ist jedoch, daß die Verfahrensparameter beim Sprühen so eingestellt sind, daß ein sauberer Auftrag auf das erste Schichtmaterial erreicht wird. Vorzugsweise wird mit einem relativ niedrigen Luftdruck, bevorzugt weniger als 10 bar, besonders bevorzugt zwischen 0,2 und 2 bar, gearbeitet. Die Sprühdüse 10a sollte vorzugsweise senkrecht auf die Bahn des ersten Schichtmaterials 11 gerichtet sein.

Wie erwähnt, ist die Menge der aufgesprühten Schmelzkleberbeschichtung 11a und der Anpreßdruck der Andrückeinrichtung 13 unter Berücksichtigung der Oberflächeneigenschaften der zu verbindenden Schichtmaterialien 11 und 14 so aufeinander abzustimmen, daß auch nach dem Andrücken des zweiten Schichtmaterials keine flüssigkeitsundurchtässige durchgängige Schmelzkleberschicht entsteht. Orientierende Angaben, in welchen Bereichen sich diese Parameter vorzugsweise bewegen, wurden in dieser Beschreibung gemacht. Auf dieser Basis lassen sie reich in Einzelfall optimieren.

Dabei ist auch die Auftragstemperatur des Schmelzklebers und dessen Abkühlung bis zum Zeitpunkt des Zusammendrückens zu berücksichtigen. Diese wird durch die Transportgeschwindigkeit des ersten Schichtmaterials und den Abstand der Auftragseinrichtung 10 von der Andrückeinrichtung 13 bestimmt. Je stärker der Schmelzkleber abgekühlt ist, desto weniger besteht die Gefahr, daß er sich beim Andrücken der zweiten Schicht 14 so stark ausbreitet, daß er einen Flüssigkeitsdurchtritt verhindert. Umgekehrt muß er aber noch ausreichend heiß sein, um eine zuverlässige Verklebung zu gewährleisten.

4

Statt des in Fig. 2 dargestellten Sprühverfahrens kann wie erwähnt auch ein Radauftragswerk oder eine Siebdruckeinrichtung verwendet werden, um die unterbrochene Beschichtung auf das erste Schichtmaterial aufzubringen.

Ein Radauftragswerk arbeitet mit einem walzenartigen Auftragsrad, welches in einem Behälter rotiert, in dem sich geschmolzener Schmelzkleber befindet. Die Walzenoberfläche enthält eingravierte Vertiefungen, deren Form und Verteilung auf der Walzenoberfläche den gewünschten Schmelzkleberbereichen entspricht. Bei der Drehung des Rades durch den Schmelzkleber wird dieser in die Vertiefungen aufgenommen. Ein überschuß wird durch ein Abstreiflineal abgestreift und die Schmelzkleberbereiche werden von dem Auftragsrad auf das saugfähige Schichtmaterial übertragen.

Da der Radauftrag von Schmelzkleber an sich bekannt ist, müssen hier keine näheren Einzelheiten angegeben werden. Bezüglich der Abstimmung der Verfahrensparameter gilt entsprechendes, wie bei dem zuvor erwähnten Sprühauftrag. Im Hinblick auf die Bedingung, daß der Schmelzkleber auch nach dem Aufbringen und Andrücken des zweiten Schichtmaterials keine durchgängige, flüssigkeitsundurchlässige Schicht bilden soll, ist dabei der Prozentsatz des von den Schmelzkleberbereichen bedecktem Anteils der Verbindungsfläche, der sich in dem oben angegebenen Rahmen bewegen soll, besonders wichtig.

Siebdruckeinrichtungen zum Aufbringen von Schmelzkleber sind ebenfalls bekannt. Sie arbeiten mit einer durchbrochenen Siebwalze, durch deren Öffnungen der Schmelzkleber auf eine unter der Siebwalze hindurchgeführte Materialbahn aufgebracht werden kann. Bezüglich der Abstimmung der Verfahrensparameter gilt hier entsprechendes wie bei dem Auftrag mit einem Radauftragswerk.

Die folgenden Beispiele dienen der weiteren Erläuterung der Erfindung:

Beispiel 1a

Ein Testträger mit dem in Figur 1 dargestellten Aufbau wird unter Verwendung folgender Materialien hergestellt:

| | |
|---|---|
| Tragschicht 2: | Hostaphan RN 350 der Kalle AG Bundesrepublik Deutschland (BRD) |
| Schmelzkleber für die Schichten 3 und 5: | EVA-Schmelzkleber "Jet-Melt 3764" der 3M Company |
| Erste saugfähige Schicht: | Faservlies VLS 3512 |
| | mit einer Stärke von 0,3 mm; Schleicher & Schüll, BRD |
| Zweite saugfähige Schicht 6 | Reaktionsschicht auf Basis eines monofilen oder multifilen, Polyestergewebes, imprägniert mit Reagenzien zur Bestimmung von Eiweiß, pH und Blut im Harn sowie von Zucker im Blut, |
| | Dicke 0,4 mm |

Der Auftrag der unterbrochenen Schmelzkleberschicht 5 erfolgte mit einem Schmelzklebersprühauftragskopf "Meltex EP 25S" unter Einhaltung folgender Verfahrensbedingungen:

| | |
|---|---|
| Auftragstemperatur des Schmelzklebersprühauftrags: | 170° C |
| Abstand Sprühdüse 10 / Andruckeinrichtung 13: | 180 mm |
| Abstand Sprühdüse 10 / erstes Schichtmaterial 11: | 25 mm |
| Anpreßdruck der Walzen 15,16 gegeneinander: | 1,0 bar |
| Luftdruck des Sprühkopfs: | 0,2 bar |
| Bahngeschwindigkeit: | 1,5 m/min |
| Klebermenge beim Sprühauftrag: | ca. 20 g/m$^2$ |

Mit diesen Verfahrensbedingungen wurde eine zuverlässige Verbindung zwischen den Schichten 4 und 6 unabhängig von der speziellen Reagenzimprägnierung der Schicht 6 erreicht. Zugleich ergab sich ein ausgezeichneter Flüssigkeitsübergang zwischen den Schichten. Dies galt für beide beschriebenen Anwendungsfälle:

- bei der Blutbestimmung wurde die Probe auf die Schicht 4 im Bereich 4a aufgegeben und von dort unter die Schicht 6 transportiert und von dieser aufgesaugt,
- bei den Harntests wurde die Probe unmittelbar auf die Schicht 6 aufgegeben, und die Schicht 4 diente als Absaugschicht zum Absaugen eines Probenüberschusses.

Beispiel 1b

Ein Testträger mit dem in Figur 1 dargestellten Aufbau wurde völlig analog zu Beispiel 1a hergestellt, wobei jedoch die saugfähige Schicht 6 aus Faservlies bzw. aus Papier mit einer Dicke von 0,4 mm bestand. Damit wurden im wesentlichen die gleichen Ergebnisse, wie zuvor beschrieben, erzielt.

Beispiel 2

Ein ähnlicher Testträgeraufbau wurde mit einem Radauftragswerk hergestellt. Das Rad war dabei mit Vertiefungen von 0,1 mm graviert, deren Fläche jeweils etwa 0,1 mm² betrug, wobei insgesamt 20% der Verbindungsfläche mit Schmelzkleber beschichtet wurde.

Verfahrensparameter

| Schmelzkleberauftragstemperatur: | 150° C |
| Andruckwalzen temperiert auf: | 40° C |
| Anpreßdruck: | 1 bar |

Auch in diesem Fall wurden ähnlich gute Ergebnisse wie mit dem Sprühauftrag erhalten.

**Patentansprüche**

1. Verfahren zur Herstellung eines diagnostischen Testträgers zur Untersuchung einer Probenflüssigkeit, welches einen Verfahrensschritt einschließt, bei dem zwei für die Probenflüssigkeit saugfähige Schichtmaterialien derart miteinander verbunden werden, daß die Probenflüssigkeit durch die Verbindungsfläche zwischen den Schichten hindurch aus der einen Schicht in die andere Schicht übertreten kann, **dadurch gekennzeichnet,** daß ein Schmelzkleber in Form einer unterbrochenen Beschichtung auf das erste Schichtmaterial aufgebracht und das zweite Schichtmaterial, während der Schmelzkleber ausreichend heiß ist um bindefähig zu sein, angedrückt wird, wobei die Menge des Schmelzklebers und der Anpreßdruck unter Berücksichtigung der Oberflächeneigenschaften der zu verbindenden Schichten so aufeinander abgestimmt sind, daß auch nach dem Aufbringen und Andrücken des zweiten Schichtmaterials keine durchgängige flüssigkeitsundurchlässige Schmelzkleberschicht entsteht.

2. Verfahren nach Anspruch 1, **dadurch gekennzeichnet,** daß der Schmelzkleber durch Aufsprühen mit Hilfe eines unter Druck stehenden Gases aufgebracht wird.

3. Verfahren nach Anspruch 1 oder 2, **dadurch gekennzeichnet,** daß die aufgebrachte Schmelzklebermenge zwischen 2 und 50 g/m², besonders bevorzugt zwischen 10 und 25g/m² liegt.

4. Verfahren nach Anspruch 1 oder 2, **dadurch gekennzeichnet,** daß das zweite Schichtmaterial mit einem Druck zwischen, 0,2 bar und 2,0 bar angedrückt wird.

5. Verfahren nach Anspruch 2, **dadurch gekennzeichnet,** daß der Schmelzkleber mit einem Gasdruck von maximal 10 bar, vorzugsweise 0,2 - 2,0 bar versprüht wird.

6. Verfahren nach Anspruch 1, **dadurch gekennzeichnet,** daß der Schmelzkleber in Form von die Vierbindungsfläche nur teilweise bedeckenden Bereichen, zwischen denen sich freie Bereiche befinden, auf das erste Schichtmaterial aufgebracht wird.

7. Verfahren nach Anspruch 6, **dadurch gekennzeichnet,** daß der Schmelzkleber im Siebdruckverfahren aufgebracht wird.

8. Verfahren nach Anspruch 6, **dadurch gekennzeichnet,** daß der Schmelzkleber mit einem Radauftragswerk aufgebracht wird.

9. Diagnostischer Testträger zur Untersuchung einer Probenflüssigkeit mit zwei saugfähigen Schichten (4,6), die derart übereinander angeordnet sind, daß die Probenflüssigkeit durch die Verbindungsfläche zwischen den Schichten hindurch aus der einen Schicht in die andere Schicht übertreten kann, **dadurch gekennzeichnet,**
daß die saugfähigen Schichten (4,6) durch eine unterbrochene, flüssigkeitsdurchlässige Schmelzkleberschicht (5) miteinander verbunden sind.

10. Testträger nach Anspruch 9, **dadurch gekennzeichnet,** daß die unterbrochene flüssigkeitsdurchlässige Schmelzkleberschicht (5) aus einem durch Aufsprühen gebildeten Gewirr von fadenförmigem Schmelzkleber besteht.

11. Testträger nach Anspruch 9, **dadurch gekennzeichnet,** daß die unterbrochene flüssigkeitsdurchlässige Schmelzkleberschicht (5) aus voneinander getrennten Schmelzkleberbereichen mit dazwischenliegenden schmelzkleberfreien Bereichen besteht.

12. Testträger nach Anspruch 11, **dadurch gekennzeichnet,** daß die Schmelzkleberbereiche zwischen 10 % und 80 % der Verbindungsfläche bedecken.

13. Testträger nach Anspruch 11, **dadurch gekennzeichnet,** daß die Schmelzkleberbereiche über die Verbindungsfläche verteilte Partikel sind.

14. Testträger nach Anspruch 13, **dadurch gekennzeichnet,** daß die Schmelzkleberpartikel im Mittel einer Flächenausdehnung von höchstens 1,0 mm$^2$ haben.

**Claims**

1. Process for the production of a diagnostic test carrier for the investigation of a sample liquid, which includes a process step in which two layer materials absorbent for the sample liquid are connected with one another in such a way that the sample liquid can pass over from one layer into the other layer through the connecting surface between the layers, characterised in that a melt adhesive is applied to the first layer material in the form of a discontinuous coating and the second layer material, while the melt is sufficiently hot in order to be bindable, is pressed on, whereby the amount of the melt adhesive and the pressing-on force are so adjusted to one another with regard to the surface properties of the layers to be bonded that, even after the application and pressing on of the second layer material, a continuous liquid-impermeable melt adhesive layer does not result.

2. Process according to claim 1, characterised in that the melt adhesive is applied by spraying on with the help of a pressurised gas.

3. Process according to claim 1 or 2, characterised in that the amount of melt adhesive applied lies between 2 and 50 g/m$^2$, especially preferably between 10 and 25 g/m$^2$.

4. Process according to claim 1 or 2, characterised in that the second layer material is pressed on with a pressure between 0.2 and 2.0 bar.

5. Process according to claim 2, characterised in that the melt adhesive is sprayed on with a gas pressure of maximum 10 bar, preferably 0.2 - 2.0 bar.

6. Process according to claim 1, characterised in that the melt adhesive is applied on the first layer material in the form of regions only partly covering the connecting surface, between which are present free regions.

7. Process according to claim 6, characterised in that the melt adhesive is applied by the screen printing process.

8. Process according to claim 6, characterised in that the melt adhesive is applied with a wheel application device.

9. Diagnostic test carrier for the investigation of a sample liquid with two absorbent layers (4, 6) which are arranged over one another in such a manner that the sample liquid can pass over through the connecting surface between the layers from one layer into the other layer, characterised in that the

7

absorbent layers (4, 6) are connected with one another by a discontinuous, liquid-permeable melt adhesive layer (5).

10. Test carrier according to claim 9, characterised in that the discontinuous liquid-permeable melt adhesive layer (5) consists of an entanglement of filamentary melt adhesive formed by spraying on.

11. Test carrier according to claim 9, characterised in that the discontinuous liquid-permeable melt adhesive layer (5) consists of melt adhesive regions separated from one another with intermediate-lying melt adhesive-free regions.

12. Test carrier according to claim 11, characterised in that the melt adhesive regions cover between 10% and 80% of the connecting surface.

13. Test carrier according to claim 11, characterised in that the melt adhesive regions are particles distributed over the connecting surface.

14. Test carrier according to claim 13, characterised in that the melt adhesive particles have, on average, a superficial extent of at most 1.0 mm$^2$.

**Revendications**

1. Procédé de préparation d'un support de test diagnostique pour la détermination d'un liquide échantillon, comprenant une étape de préparation dans laquelle deux matériaux stratifiés, ayant un pouvoir absorbant vis-à-vis du liquide échantillon, sont reliés entre eux de telle façon que le liquide échantillon puisse passer d'uns couche de matériau à l'autre à travers la surface de jonction entre les couches, caractérisé en ce qu'une colle à fusion est appliquée, sous la forme d'un revêtement discontinu, sur le premier matériau stratifié, et que le second matériau stratifié est pressé dessus tandis que la colle fusion est suffisamment chaude pour assurer la fixation, la quantité de colle à fusion et la force de compression étant ajustées l'une par rapport à l'autre, compte tenu des propriétés de surface des couches à lier entre elles, de telle façon, qu'après l'application et la compression du second matériau stratifié, il ne se forme pas une couche complète de colle à fusion inperméable aux liquides.

2. Procédé selon la revendication 1, caractérisé en ce que la colle à fusion est déposée par pulvérisation à l'aide d'un gaz sous pression.

3. Procédé selon la revendication 1 ou 2, caractérisé en ce que la quantité de colle déposée est comprise entre 2 et 50 g/m$^2$, en particulier 10 et 25 g/m$^2$

4. Procédé selon la revendication 1 ou 2, caractérisé en ce que le second matériau stratifié est soumis à une force de compression comprise entre 0,2 bar et 2,0 bar.

5. Procédé selon la revendication 2, caractérisé en ce que la colle à fusion est appliquée par pulvérisation sous une pression de gaz maximale de 10 bar, de préférence 0,2-2,0 bar.

6. Procédé selon la revendication 1, caractérisé en ce que la colle à fusion est appliquée sur le premier matériau stratifié sous la forme de zones qui ne recouvrent que partiellement la surface de jonction, et entre lesquelles se trouvent des zones libres.

7. Procédé selon la revendication 6, caractérisé en ce que la colle à fusion est appliquée par un procédé de sérigraphie.

8. Procédé selon la revendication 6, caractérisé en ce que la colle à fusion est appliquée au moyen d'une machine encolleuse à roue.

9. Support de test diagnostique pour la détermination, d'un liquide échantillon, comprenant deux couches absorbantes (4, 6) qui sont superposées de façon que le liquide échantillon puisse passer d'une couche à l'autre à travers la surface de jonction entre les couches, caractérisé en ce que les couches absorbantes (4, 6) sont reliées entre elles par une couche de colle à fusion (5) discontinue, perméable aux liquides.

**10.** Support de test selon la revendication 9, caractérisé en ce que la couche (5) de colle à fusion, discontinue, perméable aux liquides, est constituée par un enchevêtrement de la colle à fusion en forme de fibres, formé par pulvérisation.

**11.** Support de test selon la revendication 9, caractérisé en ce que la couche (5) de colle à fusion, discontinue, perméable aux liquides, est constituée par des zones de colle à fusion séparées entre elles, avec des zones dépourvues de colle à fusion situées entre les précédentes.

**12.** Support de test selon la revendication 11, caractérisé en ce que les zones de colle à fusion recouvrent entre 10 % et 80 % de la surface de jonction.

**13.** Support de test selon la revendication 11, caractérisé en ce que les zones de colle à fusion sont des parcelles séparées par la surface de jonction.

**14.** Support de test gelon la revendication 13, caractérisé en ce que les parcelles de colle à fusion présentent en moyenne une superficie mesurant au plus 1,0 mm$^2$.

Fig.1

Fig. 2

Fig. 3